(19) Europäisches Patentamt
European Patent Office
Office européen des brevets

(11) Veröffentlichungsnummer: **0 001 603**
**B1**

(12) # EUROPÄISCHE PATENTSCHRIFT

(21) Anmeldenummer: **78101098.8**

(22) Anmeldetag: **07.10.78**

(51) Int. Cl³: **C 07 C 118/00,**
C 07 C 119/042

(54) ## Verfahren zur Herstellung von Isocyanaten

(30) Priorität: **19.10.77 DE 2746963**

(43) Veröffentlichungstag der Anmeldung:
**02.05.79 Patentblatt 79/09**

(45) Bekanntmachung des Hinweises auf die Patenterteilung:
**23.07.80 Patentblatt 80/15**

(84) Benannte Vertragsstaaten:
**BE DE FR GB NL**

(56) Entgegenhaltungen:
**DE - A - 2 225 365**
**DE - B - 1 205 087**
**DE - C - 748 714**

(73) Patentinhaber: **Bayer AG**
**Zentralbereich Patente, Marken und Lizenzen**
**D - 5090 Leverkusen 1, Bayerwerk (DE)**

(72) Erfinder: **Freimuth, Reinhard, Dr.**
**Klutstein 35**
**D - 5060 Bergisch-Gladbach 2 (DE)**
**Wagner, Kuno, Dr.**
**Am Kiesberg 8**
**D - 5090 Leverkusen (DE)**
**Findeisen, Kurt, Dr.**
**In der Follmühle 10**
**D - 5068 Odenthal 2 (DE)**
**König, Klaus, Dr.**
**Heymannstrasse 50**
**D - 5090 Leverkusen (DE)**
**Heitkämper, Peter, Dr.**
**Wiedstrasse 6**
**D - 4047 Dormagen (DE)**

EP 0 001 603 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von Isocyanaten

Die vorliegende Erfindung betrifft ein Verfahren zur Herstellung von gegebenenfalls olefinisch ungesättigten Alkoxymethylisocyanaten.

Alkoxymethylisocyanate insbesondere Methoxymethylisocyanat (MMJ) sind bekannt. Diese Verbindungen sind insbesondere interessante Zwischenprodukte zur Herstellung von vernetzbaren Lackbindemitteln (vgl. z.B. DT—PSen 1 244 410, 1 619 238, 1 911 180, 1 644 801, DT—OSen 1 570 578, 1 595 701 oder 1 644 815).

Trotz der lacktechnisch hervorragenden Eigenschaften der in diesen Literaturstellen beschriebenen Bindemittel, fanden die Alkoxymethylisocyanat-Derivate bislang noch keinen Eingang in die Praxis, da bislang noch kein technisch völlig zufriedenstellender Weg zur Herstellung der Alkoxymethylisocyanate bekannt geworden ist. Das bekannte Herstellungsverfahren von Alkoxymethylisocyanaten insbesondere von Methoxymethylisocyanat (vgl. z.B. DT—AS 1 205 087) durch Umsetzung von Alkyl- chlormethyl-äthern insbesondere von Chlormethyl-methyläther mit Natriumcyanat mit dem Nachteil behaftet, daß es sich bei dem als Ausgangsmaterial einzusetzenden Chlormethyl-äther um eine Substanz handelt, die bei ihrer großtechnischen Herstellung stets mit physiologisch äußerst bedenklichen Begleitstoffen (Bis-(chlormethyl)-äther) verunreinigt ist, so daß der bekannte Weg zur Herstellung der Alkoxymethylisocyanate insbesondere zur Herstellung von Methoxymethylisocyanat großtechnisch nicht gangbar ist.

Es war somit Aufgabe der vorliegenden Erfindung, ein neues Verfahren zur Herstellung von Alkoxymethylisocyanaten insbesondere von Methoxymethylisocyanat zur Verfügung zu stellen, welches auch im großtechnischen Maßstab durchführbar ist. Diese Aufgabe konnte durch das nachstehend beschriebene erfindungsgemäße Verfahren gelöst werden.

Gegenstand der vorliegenden Erfindung ist ein Verfahren zur Herstellung von Isocyanaten der Formel

$$R—O—CH_2—NCO$$

in welcher

R für einen gesättigten aliphatischen Kohlenwasserstoffrest mit 1—4 Kohlenstoffatomen oder einen Allylrest steht, dadurch gekennzeichnet, daß man trisubstituierte Harnstoffe der Formel

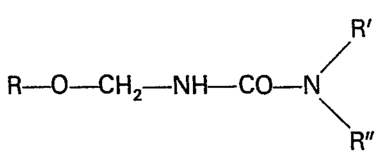

in welcher

R die bereits genannte Bedeutung hat,

R′ und R″ für beliebige, gleiche oder verschiedene, unter den Reaktionsbedingungen inerte organische Reste stehen, wobei die Reste R′ und R″ zusammen mit dem Stickstoffatom der Harnstoff-Gruppierung auch einen heterocyclischen Ring bilden können, mit organischen Mono- oder Polyisocyanaten, welche bei 1 mbar einen mindestens 10°C über dem Siedepunkt des Verfahrensprodukts liegenden Siedepunkt aufwiesen, im Temperaturbereich von 50—250°C umsetzt.

Besonders bevorzugte, beim erfindungsgemäßen Verfahren als Ausgangsmaterial einzusetzende trisubstituierte Harnstoffe sind solche der genannten Formel, für welche R für einen Methylrest steht.

Die Natur der Substituenten R′ und R″ ist für die Durchführbarkeit des erfindungsgemäßen Verfahrens von völlig untergeordneter Bedeutung. Im allgemeinen handelt es sich um geradkettige oder verzweigte, gegebenenfalls durch Halogenatome, Nitril-, Aryl- oder niedrige Alkoxygruppen substituierte Alkyloder Alkenylreste, gegebenenfalls durch Halogen; niedere Alkyl- oder Alkoxy-Gruppen substituierte Cycloalkyl oder Cycloalkenylreste, gegebenenfalls durch Halogen, Nitro-, Nitril-, Carbalkoxy-, Carbonyl-, niedere Alkyl, Alkenyl-, Alkoxy- oder Aralkyl- Gruppen substituierte Arylreste mit bis zu 16 Kohlenstoffatomen.

Besonders bevorzugt leiten sich die Reste R′ und R″ von den entsprechenden einfachen sekundären Aminen

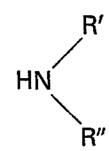

ab, dies bedeutet, daß die Reste R′ und R″ besonders bevorzugt Alkylreste mit 1—6 Kohlenstoffatomen, die gegebenenfalls ungesättigt sein oder zusammen mit dem Stickstoffatom einen gesättigten heterocyclischen gegebenenfalls ein Äther-Sauerstoffatom enthaltenden Ring bilden können, bedeuten.

Typische Vertreter von beim erfindungsgemäßen Verfahren bevorzugt einzusetzenden trisubstituierten Harnstoffe sind demnach N-Methoxymethyl-N′, N′-dimethyl-harnstoff, N-Methoxymethyl-N′, N′-diäthylharnstoff, N-Butoxymethyl-N,N′-diisopropyl-harnstoff, N-Butoxymethyl-N′,N′-dicyclohexyl-harnstoff, N-Butoxymethyl-N′,N′-diallyl-harnstoff, N-(Methoxymethyl-aminocarbonyl)-piperidin oder -p-morpholin.

Die Herstellung der genannten Ausgangsverbindungen für das erfindungsgemäße Verfahren gelingt nach an sich bekannten Verfahren des Standes der Technik durch

Umsetzung der entsprechenden disubstituierten Harnstoffe

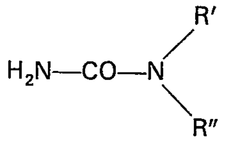

(hergestellt z.B aus dem entsprechenden sek. Amin, Phosgen und Ammoniak) mit Formaldehyd und anschließende Verätherung des dabei entstehenden N-Methylol-substituierten Harnstoffs der Formel

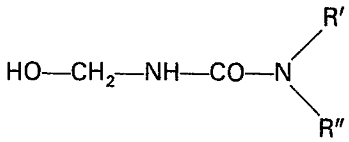

mit Alkoholen der Formel

R—OH

Dieser Syntheseweg ist im Prinzip beispielsweise in Annalen der Chemie *361,* 133, 135 (1908); Berichte der Deutschen Chemischen Gesellschaft *41,* 24 (1908); J. of the Chem. Society of Japan *11* [3], 248 (1936) oder US—PS 3 125 601 beschrieben.

Das erfindungsgemäße Verfahren wird in Gegenwart von beliebigen Isocyanatgruppen aufweisenden, ansonsten jedoch inerten, bei 1 mbar mindestens 10°C höher als die erfindungsgemäßen Verfahrensprodukte siedenden organischen Verbindungen durchgeführt. Vorzugsweise werden die höhersiedenden Hilfsisocyanate in solchen Mengen eingesetzt, daß im Reaktionsgemisch pro Mol des trisubstituierten Harnstoffs mindestens 1, vorzugsweise 1 bis 20, besonders bevorzugt 1 bis 10, insbesondere 2 bis 6 Val an Isocyanatgruppen des Hilfsisocyanats vorliegen.

Sofern die genannte Bedingung bezüglich der Siedpunktdifferenz erfüllt ist, können beim erfindungsgemäßen Verfahren beliebige obiger Definition entsprechende Isocyanate bzw. Polyisocyanate eingesetzt werden. Dies bedeutet insbesondere, daß beliebige aliphatische, cycloaliphatische, araliphatische, aromatische oder heterocyclische Monooder Polyisocyanate in Betracht kommen. Als Monoisocyanate werden beispielsweise verwendet: 6-Chlorhexylisocyanat, Dodecylisocyanat, Tetradecylisocyanat, Hexadecylisocyanat, Octadecylisocyanat, Stearylisocyanat, 4-Chlorcyclohexylisocyanat, 4-Chlorphenylisocyanat, 3,4-Dichlorphenylisocyanat, 2-Nitrophenylisocyanat, 4-Nitrophenylisocyanat, 3-Chlor-p-tolylisocyanat, 1-Naphthylisocyanat, als Polyisocyanate beispielsweise.

Äthylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato - 3,3,5 - trimethyl - 5 - isocyanatomethyl-cyclohexan (DAS 1 202 785), 2,4- und 2,6-Hexahydrotoluylendiisocyanat sowie beliebige Gemische dieser Isomeren, Hexahydri - 1,3 - und/oder -1,4 - phenylen - diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4''-triisocyanat, Polyphenyl-polymethylen-polyisocyanate, wie sie durch Anilin-Formaldehyd-Kondensation und anschließende Phosgenierung erhalten und z.B. in den britischen Patentschriften 874 430 und 848 671 beschrieben werden, perchlorierte Arylpolyisocyanate, wie sie z.B. in der deutschen Auslegeschrift 1 157 601 beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394, in der britischen Patentschrift 889 050 und in der französischen Patentschrift 7 017 514 beschrieben werden, durch Telomerisationsreaktionen hergestellte Polyisocyanate, wie sie z.B. in der belgischen Patentschrift 723 640 beschrieben werden, Estergruppen aufweisende Polyisocyanate, wie sie z.B. in den britischen Patentschriften 956 474 und 1 072 956, in der amerikanischen Patentschrift 3 567 764 und in der deutschen Patentschrift 1 231 688 genannt werden, Umsetzungsprodukte der obenganannten Isocyanate mit Acetalen gemäß der deutschen Patentschrift 1 072 385.

Es ist auch möglich, die bei der technischen Isocyanatherstellung anfallenden Isocyanatgruppen aufweisenden Destillationsrückstände, gegebenenfalls gelöst in einem oder mehreren der vorgenannten Mono- oder Polyisocyanate, einzusetzen. Ferner ist es möglich, beliebige Mischungen der vorgenannten Mono- oder Polyisocyanate zu verwenden.

Besonders bevorzugt werden in der Regel das

Chlorhexylisocyanat, sowie die technisch leicht zugänglichen Polyisocyanate, z.B. das Hexamethylendiisocyanat, das Isophorondiisocyanat, das 2,4- und 2,6-Toluylendiisocyanat sowie beliebige Gemische dieser Isomeren ("TDI"), Polyphenyl-polymethylen-polyisocyanate, wie sie durch Anilin-Formaldehyd-Kondensation und anschließende Phosgenierung hergestellt werden ("rohes MDI") und Urethangruppen, Allophanatgruppen, Isocyanuratgruppen, Harnstoffgruppen oder Biuretgruppen aufweisenden Polyisocyanate ("modifizierte Polyisocyanate") eingesetzt.

Das erfindungsgemäße Verfahren erfolgt nach dem Vermischen der trisubstituierten Harnstoffe mit dem Hilfsisocyanat durch Erhitzen auf 50—250°C, vorzugsweise 60—160°C und insbesondere 80—130°C bei einem Druck von 0,1—1013 mbar (Normaldruck) vorzugsweise 1 bis 400 mbar gegebenenfalls in Gegenwart eines Hilfslösungsmittels z.B. Chlorbenzol, Dichlorbenzol, Trichlorbenzol, Dioxan, Benzol, Toluol, Xylol, Diphenyläther oder Sulfolan. Im allgemeinen wird die Umsetzung ohne Lösungsmittel durchgeführt.

Die erfindungsgemäßen Verfahrensprodukte können aus den Reaktionsgemischen unter den angegebenen Temperatur- und Druckbedingungen im allgemeinen destillativ gewonnen werden.

Gemäß einer bevorzugten Ausführungsform des erfindungsgemäßen Verfahrens erfolgt die Umsetzung zwischen trisubstituierten Harnstoffen und Hilfsisocyanaten in Gegenwart von Carbodiimidgruppen aufweisenden organischen Verbindungen. Die Mitverwendung derartiger Carbodiimidgruppen aufweisender organischen Verbindungen führt nicht nur zu beträchtlichen Ausbeuteverbesserungen, sondern gestattet auch den Einsatz von trisubstituierten Harnstoffe geringer Reinheit, wie sie bei dem genannten Herstellungsverfahren als technische Produkte anfallen, ohne weitere Reinigung.

Als erfindungsgemäß einzusetzende Carbodiimide kommen beliebige organische Carbodiimidgruppen aufweisende Verbindungen, insbesondere aliphatische, cycloaliphatische, araliphatische oder aromatische Mono- oder Polycarbodiimide in Betracht, sofern sie keine weiteren, den Reaktionsablauf störenden Reaktivgruppen wie beispielsweise gegenüber Isocyanatgruppen reaktionsfähigen Gruppen aufweisen.

Derartige Carbodiimidgruppen aufweisenden organischen Verbindungen werden in an sich bekannter Weise durch Carbodiimidisierung von organischen Mono- oder Polyisocyanaten der oben beispielhaft genannten Art hergestellt.

Geeignete Carbodiimide sind beispielsweise die Carbodiimidisierungsprodukte von Monoisocyanaten der oben beispielhaft genannten Art wie z.B. Diäthylcarbodiimid, Dipropylcarbodiimid, Di-iso-propylcarbodiimid, Dibutylcarbodiimid, Dicyclohexylcarbodiimid, Di - 2 - methyl-cyclohexylcarbodiimid, Diphenylcarbodiimid oder die Oligocarbodiimidisierungsprodukte von Diisocyanaten wie Hexamethylendiisocyanat, 2,4 - Diisocyanatotoluol, 4,4' - Diisocyanatodiphenylmethan oder 3,3,5 - Trimethyl - 5 - isocyanatomethyl-cyclohexylisocyanat mit einem Oligomerisationsgrad von 2—15 (Oligocarbodiimid mit 2—15 Carbodiimidgruppen).

Die Carbodiimide werden beim erfindungsgemäßen Verfahren vorzugsweise in solchen Mengen eingesetzt, daß im Reaktionsgemisch für jede Harnstoffgruppe

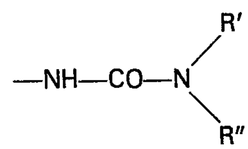

0,02 bis 0,4, vorzugsweise 0,03 bis 0,2 Mol, Carbodiimidgruppen

—N=C=N—

vorliegen, was in Abhängigkeit von der Größe der indifferenten Reste sowohl der Harnstoff-Ausgangsmaterialien als auch der Carbodiimide einer Menge von ca. 3—50 vorzugsweise 5—20 Gew.—% Carbodiimid bezogen auf den eingesetzten trisubstituierten Harnstoff entspricht.

Falls als Carbodiimidgruppen aufweisende organische Verbindungen solche verwendet werden, welche neben den Carbodiimidgruppen auch noch freie Isocyanatgruppen aufweisen (Carbodiimidisierungsprodukte von Di- und/oder Polyisocyanaten), ist es möglich, auf die Mitverwendung der oben beispielhaft genannten Hilfsisocyanate ganz oder teilweise zu verzichten, da in einem solchen Falle die freien Isocyanatgruppen der Carbodiimide die Rolle des Hilfsisocyanats übernehmen. Es ist in diesem Falle darauf zu achten, daß die Gesamtmenge der im Reaktionsgemisch vorliegenden Isocyanatgruppen den oben gemachten Ausführungen entspricht, und daß das Isocyanatgruppen aufweisende Carbodiimid bei 1 mbar einer mindestens 10°C oberhalb des Siedepunkts des Verfahrensprodukts liegenden Siedepunkt aufweist, eine Bedingung, die im Falle der Carbodiimidgruppen aufweisenden Polyisocyanate ohnehin erfüllt ist, da es sich im allgemeinen um nicht destillierbare Substanzen handelt.

Im Falle der Mitverwendung der Carbodiimidgruppen aufweisenden organischen Verbindungen wird das erfindungsgemäße Verfahren vorzugsweise in der Weise durchgeführt, daß man den zu spaltenden trisubstituierten Harnstoff mit Carbodiimid versetzt, das höhersiedende Mono- oder Diisocyanat hinzufügt und das erfindungsgemäß entstehende Isocyanat isoliert oder das Reaktionsgemisch direkt zur Umsetzung nach dem Isocyanat-Polyadditionsverfahren verwendet.

Beispielsweise gibt man zu einem Gemisch aus N,N-Dimethyl-N'-methoxymethyl-harnstoff und Diphenylcarbodiimid, das einige Zeit bei 50°C belassen worden war, Hexamethylendiisocyanat unter vermindertem Druck bei 100°C, beläßt noch kurze Zeit bei 120°C und isoliert dann in einer gekühlten Vorlage das Methoxymethylisocyanat.

Das erfindungsgemäße Verfahren gestattet die Herstellung von Alkoxymethylisocyanaten, insbesondere von Methoxymethylisocyanat, für welches bislang noch keine technisch völlig zufriedenstellende Herstellungsweise bekannt geworden war.

Beispiel 1

In einem 1 l-Dreihalskolben, der mit Rührer, Destillationsbrücke mit Trockeneis-gekühlter Vorlage und Tropftrichter ausgerüstet ist, werden 132 g (1 Mol) technischer N, N-Dimethyl-N'-methoxymethylharnstoff mit 13 g Diphenylcarbodiimid während 2 h auf 50°C erwärmt. Nach Zugabe von 504 g (3 Mol) Hexamethylendiisocyanat des Siedepunkts 140°C/20 mbar erhält man bei 80—120°C unter einem verminderten Druck von 20 mbar in der gekühlten Vorlage nach 2 h 80 g (91%) Methoxymethylisocyanat (Siedepunkt: 90°C/1013 mbar).

*Vergleichsbeispiel*

Analog zum Verfahren von Beispiel 1, nur ohne Zugabe von Carbodiimid, wird Methoxymethylisocyanat nur in Spuren isoliert, offensichtlich wegen der beträchtlichen Mengen an Verunreinigungen im eingesetzten Harnstoffderivat.

Beispiel 2

In der Apparatur des Beispiels 1 werden 160 g (1 Mol) technischer N,N-Diäthyl-N'-methoxymethylharnstoff mit 16 g Diphenylcarbodiimid während 2 Stunden auf 50°C erwärmt. Nach Zugabe von 504 g (3 Mol) Hexamethylendiisocyanat erhält man bei 80—120°C unter einem vermindertem Druck von 20 mbar in der gekühlten Vorlage nach 2 h 76,5 g (88%) Methoxymethylisocyanat.

*Vergleichsbeispiel*

Analog zum Verfahren von Beispiel 2, nur ohne Zugabe von Carbodiimid, wird Methoxymethylisocyanat nur in Spuren isoliert, offensichtlich wegen der beträchtlichen Mengen an Verunreinigungen im eingesetzten Harnstoffderivat.

Beispiel 3

Analog Beispiel 1, d.h. unter Verwendung der gleichen Apparatur und der gleichen Äquivalenzverhältnisse Harnstoff:Hilfsisocyanat wird aus technischem N, N-Dicyclohexyl-N'-i-butoxymethylharnstoff mit 13 Gew.-% Dicyclohexylcarbodiimid, bezogen auf den Harnstoff, bei 90—120°C und 20 mbar mit einem 80:20 Gemisch aus 2,4-und 2,6-Toluylendiisocyanat, (Siedepunkt: 127°C/16 mbar) i-Butoxymethylisocyanat (Siedpunkt: 41°C/16 mbar) in 85%iger Ausbeute gewonnen.

Beispiel 4

Analog Beispiel 1, d.h. unter Verwendung der gleichen Apparatur und der gleichen Äquivalenzverhältnisse Harnstoff:Hilfsisocyanat wird aus technischem N-Phenyl-N-methyl-N'-n-propoxymethylharnstoff mit 15 Gew.-%, bezogen auf Harnstoff, 2,2', 3,3'-Tetrachlor-diphenylcarbodiimid bei 100—130°C und 20 mbar mit einem Polyisocyanatgemisch der Diphenylmethanreihe eines NCO-Gehalts von 32%, eines Gehalts an Diisocyanate-diphenylmethan-Isomeren von ca. 60% und einem Siedepunkt von 146°C/0,7 mbar n-Propoxymethylisocyanat (Siedepunkt: 35°C/16 mbar) in 86%iger Ausbeute gewonnen.

Beispiel 5

Analog Beispiel 1, d.h. unter Verwendung der gleichen Apparatur und der gleichen Äquivalenzverhältnisse Harnstoff:Hilfsisocyanat wird aus technischem N-Benzyl-N-phenyl-N'-i-butoxymethylharnstoff mit 15 Gew.-%, bezogen auf Harnstoff, 2,2',3,3',-Tetrachlor-diphenylcarbodiimid bei 100—130°C und 20 mbar mit dem Polyisocyanatgemisch aus Beispiel 4 i-Butoxymethylisocyanat (Siedepunkt 41°C/16 mbar) in 84%iger Ausbeute gewonnen.

Beispiel 6

Analog Beispiel 1, d.h. unter Verwendung der gleichen Apparatur und der gleichen Äquivalenzverhältnisse Harnstoff:Hilfsisocyanat wird aus technischem N,N-Dihexahydrobenzyl-N'-i-butoxymethylharnstoff mit 15 Gew.-%, bezogen auf Harnstoff, 2,2',4,4'-Tetrachlor-diphenylcarbodiimid bei 90—120°C und 20 mbar mit 6-Chlorhexylisocyanat (Siedepunkt: 90°C/7 mbar) i-Butoxymethylisocyanat (Siedepunkt: 41°C/16 mbar) in 86%iger Ausbeute gewonnen.

Beispiel 7

Analog Beispiel 1, d.h. unter Verwendung der gleichen Apparatur und der gleichen Äquivalenzverhältnisse Harnstoff:Hilfsisocyanat wird aus technischem N,N-Diallyl-N'-i-butoxymethylharnstoff mit 15 Gew.-%, bezogen auf Harnstoff, 2,2',4,4'-Tetrachlor-diphenylcarbodiimid bei 90—120°C und 20 mbar mit 6-Chlorhexylisocyanat (Siedepunkt: 90°C/7 mbar) i-Butoxymethylisocyanat in 86%iger Ausbeute gewonnen.

Beispiel 8

In der Apparatur des Beispiels 1 werden 202 g (1 Mol) technischer N,N-Diäthyl-N'-i-butoxymethylharnstoff bei 60°C mit 600 g eines durch Carbodiimidisierung von Hexamethylendiisocyanat gewonnenen, nicht destillierbaren Diisocyanatocarbodiimids mit einem NCO-Gehalt

von 20 Gew.-% (vgl. Angewandte Chemie *74,* 801 (1962)) vermischt. Das Gemisch wird anschließend bei einem Druck von 20 mbar auf 100—130°C erhitzt, wobei das sich bildende i-Butoxymethylisocyanat abzudestillieren beginnt. Es werden 104 g (81%) des Monoisocyanats erhalten.

Beispiel 9

Ein 21-Dreihalskolben wurde mit Rührer, Thermometer und einer 30 cm-Füllkörperkolonne (Raschig-Ringe) versehen. Der Kolonnenkopf wurde über eine Destillationsbrücke mit einer 500 ml-Vorlage verbunden. Die Apparatur wurde über eine Trockeneis-Kühlfalle an eine Wasserstrahl-Pumpe angeschlossen. In den Reaktionskolben wurden 160 g (1 Mol) N,N-Diäthyl-N'-methoxymethyl-harnstoff (Reinheit > 98%) und 504 g (3 Mol) Hexamethylendiisocyanat eingewogen. Die Vorlage wurde mit einer ausgewogenen Menge an destilliertem Chlorbenzol (ca. 200 ml) beschickt und dann mit Trockeneis tiefgekühlt. Nachdem die Apparatur mit vollem Wasserstrahlvakuum versehen war, wurde der Reaktionskolben rasch auf 100°C, dann langsam bis auf 130°C erwärmt. Nach 4 Stunden wurde die Reaktion abgebrochen, die Vorlage nach Erwärmen auf Raumtemperatur zurückgewogen und die gewonnene chlorbenzolische MMI-Lösung auf ihren NCO-Gehalt titriert (Rohausbeuten an MMI: 87% der Theorie).

Die chlorbenzolische Lösung wurde unter Normaldruck über eine 30 cm-Füllkörperkolonne (Raschig-Ringe) destilliert und ergab praktisch reines MMI (entsprechend dem titrierten NCO-Gehalt) Reinausbeute: 80% der Theorie (bezogen auf eingesetzten Harnstoff).

Beispiel 10

Ein 21-Dreihalskolben wurde mit Rührer, Thermometer und einer 30 cm-Füllkörperkolonne (Raschig-Ringe) versehen. Der Kolonnenkopf wurde über eine Destillationsbrücke mit einer 500 ml-Vorlage verbunden. Die Apparatur wurde über eine Trockeneis-Kühlfalle an eine Wasserstrahl-Pumpe angeschlossen. In den Reaktionskolben wurden 160 g (1 Mol) N,N-Diäthyl-N'-methoxymethyl-harnstoff (Reinheit > 98%) 1 Mol) Hexamethylendiisocyanat eingewogen. Die Vorlage wurde mit einer ausgewogenen Menge an destilliertem Chlorbenzol (ca. 200 ml) beschickt und dann mit Trockeneis tiefgekühlt. Nachdem die Apparatur mit vollem Wasserstrahlvakuum versehen war, wurde der Reaktionskolben rasch auf 100°C, dann langsam bif auf 130°C erwärmt. Nach 3 Stunden wurde die Reaktion abgebrochen, die Vorlage nach Erwärmen auf Raumtemperatur zurückgewogen und die gewonnene chlorbenzolische MMI-Lösung auf ihren NCO-Gehalt titriert (Rohausbeuten an MMI: 78% der Theorie).

Die benzolische Lösung wurde unter Normaldruck über eine 30 cm-Füllkörperkolonne (Raschig-Ringe) destilliert und ergab praktisch reines MMI (entsprechend dem titrierten NCO-Gehalt) 75% der Theorie (bezogen auf eingesetzten Harnstoff).

Beispiel 11

Ein 21-Dreihalskolben wurde mit Rührer, Thermometer und einer 30 cm-Füllkörperkolonne (Raschig-Ringe) versehen. Der Kolonnenkopf wurde über eine Destillationsbrücke mit einer 500 ml-Vorlage verbunden. Die Apparatur wurde über eine Trockeneis-Kühlfalle an eine Wasserstrahlt-Pumpe angeschlossen. In den Reaktionskolben wurden 320 g (2 Mol) N,N - Diäthyl - N' - methoxymethyl-harnstoff (Reinheit > 98%) und 185 g (1,1 Mol) Hexamethylendiisocyanat eingewogen. Die Vorlage wurde mit einer ausgewogenen Menge an destilliertem Chlorbenzol (ca. 200 ml) beschickt und dann mit Trockeneis tiefgekühlt. Nachdem die Apparatur mit vollem Wasserstrahlvakuum versehen war, wurde der Reaktionskolben rasch auf 100°C, dann langsam bis auf 150°C erwärmt. Nach 2 Stunden wurde die Reaktion abgebrochen, die Vorlage nach Erwärmen auf Raumtemperatur zurückgewogen und die gewonnene chlorbenzolische MMI-Lösung auf ihren NCO-Gehalt titriert (Rohausbeuten an MMI: 74% der Theorie).

Die chlorbenzolische Lösung wurde unter Normaldruck über eine 30 cm-Füllkörperkolonne (Raschig-Ringe) destilliert und ergab praktisch reines MMI (entsprechen dem titrierten NCO-Gehalt) Reinausbeute: 52% der Theorie (bezogen auf eingesetzten Harnstoff).

Beispiel 12

Ein 21-Dreihalskolben wurde mit Rührer, Thermometer und einder 30 cm-Füllkörperkolonne (Raschig-Ringe) versehen. Der Kolonnenkopf wurde über eine Destillationsbrücke mit einer 500 ml-Vorlage verbunden. Die Apparatur wurde über eine Trockeneis-Kühlfalle an eine Wasserstrahl-Pumpe angeschlossen. In den Reaktionskolben wurden 216 g (1 Mol) N,N-Di-n-butyl-N'-methoxy-methylharnstoff (Reinheit 98%) und 168 g (1 Mol) Hexamethylendiisocyanat eingewogen. Die Vorlage wurde mit einer ausgewogenen Menge an destilliertem Chlorbenzol (ca. 200 ml) beschickt und dann mit Trockeneis tiefgekühlt. Nachdem die Apparatur mit vollem Wasserstrahlvakuum versehen war, wurde der Reaktionskolben rasch auf 100°C, dann langsam bis auf 150°C erwärmt. Nach 2 Stunden wurde die Reaktion abgebrochen, die Vorlage nach Erwärmen auf Raumtemperatur zurückgewogen und die gewonnene chlorbenzolische MMI-Lösung auf ihren NCO-Gehalt titriert (Rohausbeuten an MMI: 84 % der Theorie).

Die chlorbenzolische Lösung wurde unter Normaldruck über eine 30 cm-Füllkörperkolonne (Raschig-Ringe) destilliert und ergab praktisch reines MMI (entsprechend dem titrierten NCO-Gehalt) Reinausbeute: 79% der

Theorie (bezogen auf eingesetzten Harnstoff).

**Patentansprüche**

1. Verfahren zur Herstellung von Isocyanaten der Formel

$$R—O—CH_2—NCO$$

in welcher

R für einen gesättigten aliphatischen Kohlenwasserstoffrest mit 1—4 Kohlenstoffatomen oder einen Allylrest steht, dadurch gekennzeichnet, daß man trisubstituierte Harnstoffe der Formel

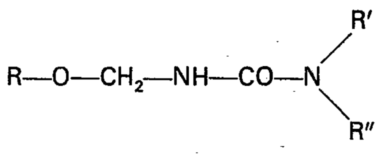

in welcher

R die bereits genannte Bedeutung hat,

R′ und R″ für beliebige, gleiche oder verschiedene, unter den Reaktionsbedingungen inerte organische Reste stehen, wobei die R′ und R″ zusammen mit dem Stickstoffatom der Harnstoff-Gruppierung auch einen heterocyclischen Ring bilden können, mit organischen Mono- oder Polyisocyanaten, welche bei 1 mbar einen mindestens 10°C über dem Siedepunkt des Verfahrensprodukts liegenden Siedepunkt aufweisen, im Temperaturbereich von 50—250°C umsetzt.

2. Verfahren gemäß Anspruch 1, dadurch gekennzeichnet, daß man die Umsetzung in Gegenwart von Carbodiimidgruppen aufweisenden organischen Verbindungen durchführt.

3. Ausführungsform des Verfahrens gemäß Anspruch 2, dadurch gekennzeichnet, daß man anstelle oder neben der gleichzeitigen Verwendung von höhersiedenden Mono- oder Polyisocyanaten und Carbodiimidgruppen aufweisenden organischen Verbindungen, Carbodiimidgruppen aufweisende organische Isocyanate einsetzt, welche bei 1 mbar einen mindestens 10°C über dem Siedepunkt des Verfahrensprodukts liegenden Siedepunkt aufweisen.

**Revendications**

1. Procédé pour la préparation d'isocyanates répondant à la formule

$$R—O—CH_2—NCO$$

dans laquelle

R représente un reste hydrocarboné aliphatique saturé en $C_1$—$C_4$ ou un reste allyle,
ce procédé se caractérisant en ce que l'on fait réagir dans un intervalle de température de 50 à 250°C des urées trisubstituées répondant à la formule

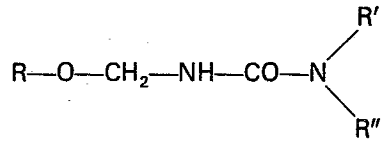

dans laquelle

R a la signification indiquée ci-dessus,

R′ et R″ représentent des restes organiques quelconques, identiques ou différents, inertes dans les conditions de la réaction, R′ et R″ pouvant également former, avec l'atome d'azote du groupement urée, un hétérocycle,
avec des mono- ou poly-isocyanates organiques dont le point d'ébullition sous une pression de 1 millibar est supérieur d'au moins 10°C au point d'ébullition du produit de réaction.

2. Procédé selon la revendication 1, caractérisé en ce que l'on effectue la réaction en présence de composés organiques portant des groupes carbodiimide.

3. Mode de réalisation du procédé selon la revendication 2, caractérisé en ce que, à la place des mono- ou poly-isocyanates à point d'ébullition plus élevé et des composés organiques portant des groupes carbodiimide, ou avec ces composés, on utilise des isocyanates organiques portant des groupes carbodiimide et qui ont un point d'ébullition sous une pression de 1 millibar supérieur d'au moins 10°C à celui du produit de réaction.

**Claims**

1. Process for the preparation of isocyanates of the formula:

$$R—O—CH_2—NCO$$

in which

R represents a saturated aliphatic hydrocarbon radical having from 1 to 4 carbon atoms or an allyl radical,
characterised in that tri-substituted ureas of the formula:

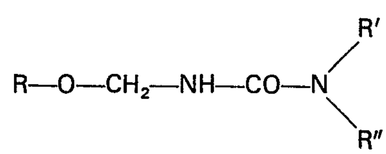

in which

R has the meaning already mentioned,

R′ and R″ represent any desired, identical or different, organic radicals which are inert under the reaction conditions, and the radicals R′ and R″ may, together with the nitrogen atom of the urea group, also form a heterocyclic ring
are reacted at temperatures of from 50 to 250°C with organic mono- or poly-isocyanates which at 1 mbar have a boiling point which is at

least 10°C above the boiling point of the product of the process.

2. Process according to claim 1, characterised in that the reaction is carried out in the presence of organic compounds which contain carbodiimide groups.

3. Embodiment of the process according to claim 2, characterised in that instead of or in addition to the simultaneous use of higher boiling mono- or poly-isocyanates and organic compounds which contain carbodiimide groups there are used carbodiimide-group-containing organic isocyanates which, at 1 mbar, have a boiling point at least 10°C above the boiling point of the product of the process.